**Europäisches Patentamt**

**European Patent Office**

**Office européen des brevets**

(19)

(11) Veröffentlichungsnummer: **0 001 443**
**B1**

# (12) EUROPÄISCHE PATENTSCHRIFT

(45) Veröffentlichungstag der Patentschrift: **24.03.82**

(21) Anmeldenummer: **78101046.7**

(22) Anmeldetag: **30.09.78**

(51) Int. Cl.³: **A 61 K 37/02,**
**A 61 K 39/00, C 07 G 7/00**

(54) Mittel zur Konzeptionsverhütung und Schwangerschaftsunterbrechung bei Primaten und Verfahren zu ihrer Herstellung.

(30) Priorität: **11.10.77 DE 2745680**

(43) Veröffentlichungstag der Anmeldung:
**18.04.79 Patentblatt 79/8**

(45) Bekanntmachung des Hinweises auf die Patenterteilung:
**24.03.82 Patentblatt 82/12**

(84) Benannte Vertragsstaaten:
**DE FR GB**

(56) Entgegenhaltungen:
**DE - A - 2 345 953**
**DE - A - 2 616 984**

(73) Patentinhaber: **BEHRINGWERKE**
**AKTIENGESELLSCHAFT**
**Postfach 1140**
**D-3550 Marburg/Lahn (DE)**

(72) Erfinder: **Bohn, Hans, Dr.**
**Oberer Eichweg 26**
**D-3550 Marburg/Lahn (DE)**
Erfinder: **Weinmann, Ernst, Dr.**
**Sonnenweg 19**
**D-3550 Marburg/Lahn (DE)**

(74) Vertreter: **Meyer-Dulheuer, Karl-Hermann, Dr.**
**et al,**
**HOECHST Aktiengesellschaft Zentrale**
**Patentabteilung Postfach 80 03 20**
**D-6230 Frankfurt/Main 80 (DE)**

Courier Press, Leamington Spa, England.

Mittel zur Konzeptionsverhütung und Schwangerschaftsunterbrechung bei Primaten und Verfahren zu ihrer Herstellung

Gegenstand der Erfindung sind Mittel zur Konzeptionsverhütung und zur schwangerschaftsunterbrechung bei Primaten auf immunologischer Basis und Verfahren zu ihrer Herstellung.

Kontrazeptive Mittel, welche auf der Grundlage immunologischer Reaktionen wirksam sind, wurden mehrfach beschrieben. So ist schon versucht worden, durch Antiseren gegen Extrakte tierischer Plazenten bei Kaninchen konzeption zu verhüten und bei Affen und Mässen Fehlgeburten herbeizuführen. In dem schwangerschaftsspezifischen $\beta_1$-Glykoprotein wurde ein wirksames Mittel zur Konzeptionsverhütung und zur Schwangerschaftsunterbrechung bei Primaten gefunden (vgl. DOS 23 45 953). Ein Antigen zur Immunologischen Fruchtbarkeitskonzrolle, bestehend aus proteinartigen Fortpflanzungshormonen, ist in DOS 26 43 023 beschrieben, ein kontrazeptiver Impfstoff in DOS 26 18 546 mit einem ähnlichen Aufbau. Insbesondere bei den letztgenannten wird als Mangel angesehen, daß antihormone eine über die Fortpflanzungsfähigkeit hinausgehende Wirksamkeit besitzen, die nicht erwünscht ist. Auch die auf der Basis der DOS 23 45 953 aufgebauten Mittel haben nicht immer die immunologische Wirksamkeit gezeigt, wie sie für eine erfolgreiche Konzeptionsregulierung erforderlich erscheint. Es hat deshalb nicht an Versuchen gefehlt, die immunogene Wirksamkeit der Antigene zu verbessern (vgl. DOS 25 43 994). Auch die DOS 26 46 223 und 25 18 546 bedienen sich modifizierter Hormonantigene.

Neben den von H. Bohn in Arch. Gynäk., 212, S. 165—175 (1972) beschriebenen löslichen Antigenen der menschlichen Plazenta sind als Inhaltsstoffe dieses Organs die Proteohormone Choriongonaditropin, Plazentalactogen und Chorionthyrotropin beschrieben, ferner die Enzyme Cystinaminopeptidase, hitzestabile alkalische Phosphatase und 17-$\beta$-Hydroxisteroid-Dehydrogenase. Über die Eignung des Schwangerschaftesspezifischen $\beta_1$-Glykoproteins als Bestandteil eines Kontrazeptiven Impfstoffes wurde bereits eingangs berichtet. Ebenso über die Verwendung der betreffenden Hormone in diesem Sinne.

Es wurden nun überraschend befunden, daß immunologisch zur Antikörperinduktion gegen das plazentenspezifische Glycoprotein PP5 befähigte Substanzen hervorragende immunologische Antikonzeptiva darstellen. Das PP5 ist ein bereits beschriebenes Placentaprotein. Es ist gemäß einem in der DOS 26 16 984 beschriebenen Verfahren erhältlich. Es zeigt eine elektrophoretische Wanderung in Agargel im Bereich der $\beta_1$-Globuline. In einem Polyacrylamidgel wandert es im Vergleich zu Albumin=100 mit einer relativem Beweglichkeit von 75. Aufgrund seines Verhaltens bei der Gelfiltration auf quervernetztem Dextran wurde ein Molekulargewicht für PP5 von etwa 50.000 abgeleitet. Am einfachsten wird es durch seine immunologische Reaktion mit einem Spezifischen Anti-PP5-Serum charakterisiert.

Gegenstand der Erfindung ist demnach ein Mittel zur Konzeptionsverhütung und zur Schwangerschaftsunterbrechung und Herbeiführung von Fehlgeburten bei Primaten, insbesondere Menschen, dadurch gekennzeichnet, daß es als wesentlichen Bestandteil eine wirksame Menge einer Substanz enthält, welche bei parenteraler Verabreichung mit dem Protein PP5 immunologisch zu reagieren oder immunologisch reaktive Substanzen, Antikörper, die gegen das PP5 gerichtet sind, zu induzieren vermag. Zunächst sind hierzu zu rechnen Antikörper mit immunologischen Determinaten, die gegen das PP5 gerichtet sind, vorzugsweise das Anti-PP5 selbst, aber auch Antikörper, welche mit Derivaten des PP5 erhalten worden sind. Von den Substanzen, welche Antikörper gegen das PP5 zu induzieren vermögen, sind an erster Stelle das PP5 zu nennen, ferner die davon abgeleiteten Derivate. Von den Derivaten des PP5 haben insbesondere solche Bedeutung, welche durch einfache Umsetzung des PP5 mit einer Proteine modifizierenden Verbindung herstellbar sind sowie die PP5-Konjugate mit Haptenen.

Bei derartigen Umsetzungen ist von zwei Grundprinzipien auszugehen. Sie führen beide zur Veränderung der nativen Struktur und damit zu einer Denaturierung des Proteins:

1. Verfahren zur chemischen Veränderung des PP5 mit Hilfe von Reaganzien, welche zu einer Veränderung vorhandener chemischer Gruppen führen.
2. Verfahren, die zu einer chemischen Veränderung des PP5 durch Einführung neuer Grippen in das Molekül bzw. die zur Verbindung des Moleküls mit niedrig- order hochmolekularen Verbindungen führen.

Zu den genannten Verfahren sind diejenigen zu zählen, die in Proteinen eine oder mehrere der folgenden Gruppen zu verändern vermögen: Amino, Guanidyl, Imidazol, Indol, aliphatisches Hydroxyl, Amid, Thioäther, Disulfid, Sulhydryl, Phenol und Carboxyl. Hierfür ist eine Reihe von Reaktionen aus der Literatur bekannt, zu denen hauptsächlich die nachfolgend genannten Reagenzien unter den beispielhaft erwähnten Bedingungen einzusetzen sind.

1.1 Oxydation mit Jodosobenzoat, Porphyrindin, Ferrocyanid oder Jod, wobei in der Regel eine Konzentration des Oxydationsmittels von 0,001—0,01 M, ein pH-Wert von 7, eine Temperatur von 0—25°C und eine Umsetzungsdauer von 5—30 Minuten anzuwenden sind. Die Oxydation mit Jod wird in Gegenwart von Jodid in hoher Konzentration bei einem pH-Wert von

**0 001 443**

1—7 ausgeführt. Die Oxydation kann auch mit Wasserstoffperoxyd vorgenommen werden. Die bevorzugten Begingungen sind dann:

Konzentration des Oxydationsmittels etwa 0,005 M, pH-Wert etwa 6,6, Temperatur etwa 25° und Reaktionsdauer 0,5—40 Stunden.

1.2 Reduktion mit Cystein, Thioglykolsäure, Thioglykol, Cyanid oder Sulfid, vorzugsweise unter folgenden Bedingungen: Konzentration des Reduktionsmittels 0,001 bis 0,1 M, pH-Wert 7— 8, Temperatur etw 25°C und Reaktionsdauer 0,1—4 Stunden.

Die Reaktionsbedingungen können im einzelnen der Fachliterature entnommen werden. Sie sind beispielsweise referiert von H. S. Olcott und H. Fraenkel-Conrat, Chem. Rev. 41, S. 151 ff (1974). Zudem ist ein Teil der Reagenzien und der Verfahrensbedingungen aus H. E. Schultze und J. F. Heremans, Molecular Biology of Human Proteins (1966), S. 40—41 und den darin auf S. 55 ff. angegebenen Referenzen zu ersehen.

Mit der Einführung neurer Gruppen in das $PP_5$ werden Derivate hergestellt, die vom immunologischen Standpunkt aus in der Regel als Hapten-Verbindungen bezeichnet werden. Nach einer anerkannten Definition ist ein "Hapten" eine proteinfreie Substanz, die mit einem Spezifischen gegen seine Konfiguration gerichteten Antikörper reagieren kann, die ihrerseits jedoch nicht zur Bildung nachweisbarer Antikörpermengen befähigt ist. Ein Hapten Bewirkt eine Immunantwort nur in Verbindung mit einem Trägerprotein. Die meisten Haptene sind niedermolekulare Verbindungen mit einem Molekulargewicht <1000. Es werden jedoch auch Makromoleküle wie beispielsweise Pneumokokkenpolysaccharide als Haptene angesehen. Eine Hapten-Protein-Verbindung führt in der Regel zur Induktion von zwei Typen von Antikörpern, wobei Spezifitäten gegen die Haptengruppierung und gegen den Proteinträger entstehen. Im Falle des $PP_5$ gilt, daß dessen Hapten-Verbindungen auch gegen das Trägerprotein im homologen System Antikörper induzieren.

Als Haptene im Sinne der Erfindung kommen die in der Literatur als solche beschriebenen chemischen Gruppen und Verbindungen in Frage. Insbesondere gehören hierzu aromatische Ringsysteme, Steroide, Peptide, Purine, Pyrimidine, Penicillin und dessen Derivate, aber auch Einzelmoleküle wie Jod, und entsprechend einer hier vorgenommenen erweiterten Definition der an den Träger von $PP_5$ zu bindenden Substanzen auch hochmolekulare Körper mit Peptid- bzw. Protein- und Kohlenhydratcharakter, die ihrerseits selbst antigene Eigenschaften haben.

Die Verfahren zur Herstellung der Derivate des $PP_5$ nach dem genannten Grundprinzip (2.) unter Verwendung von Haptenen sind als allgemein anwendbare Reaktionen für die Einführung von Proteine modifizierenden chemischen Gruppen und von Haptenen in Eiweißkörpern unter Ausbildung kovalenter Verknüpfung zwischen chemischen Substanzen und Proteinen bekannt. Insbesondere handelt es sich dabei um Reaktionen, die für die Modifikation einzelner Gruppen in Proteinmolekülen beschrieben sind. Auch diese Reaktionen sind in den Arbeiten von Olcott und Fraenkel-Conrat und von Schultze-Heremans beschrieben Beispiele solcher Verfahren sind:

2.1 Die Alkylierung mit
Jodacetat, Jodacetamid, (0,05—0,1 M, pH 7—8, 0—25°C, 0,5—2 Stunden) oder Dinitrofluorobenzol (0,17 M, pH 7—8, 25°, 2 Stunden).
2.2 Die Acylierung mit
Keten (pH 5—8, 0—25°, 5—30 Minuten),
Essigsäureanhydrid (pH 7—8, 0°, 30 Minuten),
Kohlenstoffsuboxid (pH 5—8, 0—25°, 5—30 Minuten),
Aziden, Benzoyl-, Carbobenzoxy- oder Benzolsulfonylchlorid (pH 7—9, 0—25°, 0,5—2 Stunden),
Salpetriger Säure (1M, pH 4, 30 Minuten),
Jod (pH 5—11, −5°—25°, 0,5—3 Stunden im Gegensatz zu oben beschriebener Oxydation mit geringerer Jodmenge und niedrigerer (Jodidkonzentration),
Formaldehyd (1—2 M, 25°, bei pH 7—8, 1 Stunde, bei pH 11,1—4 Tage),
Epoxiden (1—2 M, pH 5—6, 10 Minuten),
Senfgas (pH 5—6, 25°, 0,5—4 Stunden),
Säure/Alkohol (Mineralsäure in absolutem Alkohol) (0,01—0,1 M, 0—25°, 1—2 Tage),
Methyldiazoacetat, Diazoacetamid (pH 5 (Ester), pH 6 (Amid), 0°),
p-chloromercuribenzoat ($10^{-5}$—$10^{-2}$ M, pH 7, 25°, 5—30 Minuten),
Diazonium-Verbindungen (pH 7—9, 25°, 30 Min.) oder
o-Methylisoharnstoff (0,5 M, pH 10, 5, 0°, 3 Tage).

Für einige der obengenannten Gruppen wird nachfolgend auf die Verfahren näher eingegangen:

a) Jodierung
(Literatur: Kabat and Mayer's Experimental Immunochemistry, sec. Edition 1961, 816—818)

3

Jod wird als Jodanion mit Tyrosinresten des Proteins zur Umsetzung gebracht, wobei ein oder zwei Jodatome in das Tyrosinmolekül eingeführt werden und das Jodprotein entsteht. In gleicher Weise kann die Jod-Verbindung des $PP_5$ hergestellt werden.

b) Diazotierung und Kupplung
(Literatur: Kabat and Mayer's Experimental Immunochemistry, sec. Edition 1961, 816—818)

Hierzu wird eine Verbindung mit einer aromatischen Aminogruppe, beispielsweise die Arsanilsäure oder Sulfanilsäure, diazotiert und die Diazoniumverbindung sodann mit dem Protein umgesetzt. Dabei gehen vornehmlich Tyrosinreste, aber auch Histidin- und Lysinreste des Proteins eine kovalente Verbindung mit der aromatischen Komponente ein. Nach diesem Verfahren kann die Diazo-Arsanilsäure- oder die Diazobenzol-Sulfonsäure-Verbindung des $PP_5$ hergestellt werden.

c) Umsetzung mit Vinylsulfonverbindungen
(in Analogie zur Reaktion von Reaktivfarbstoffen, die als Reaktivkomponente die Vinylsulfongruppe enthalten, mit Zellulose oder Wolle)

Aliphatische oder atomatische Vinylsulfonderivate und Schwefelsäurehalbester von $\beta$-Hydroxyäthylsulfonen können mit den Aminogruppen und/oder Hydroxylgruppen des Proteins zur Reaktion gebracht werden. Auf diese Weise kann z.B. die Hapten-Verbindung des $PP_5$ mit 1-Aminobenzol-4$\beta$-Hydroxyäthylsulfon-Schwefelsäure erhalten werden.

d) Reaktion mit Isocyanat- und Isothiocyanat-Vergindungen
(Literatur: Kabat und Mayer's Experimental Immunochemistry, sec. edition 1961, 809—811)

Diese Reaktion, die über die freien Aminogruppen des Proteins abläuft, erlaubt on Analogie zu den bekannten Verfahren der Proteinchemie die Umsetzung von Haptenen mit der entsprechenden chemischen Gruppierung auch mit dem $PP_5$.

e) Dinitrophenylierung
(Literatur: Carsten, M. E.; Eisen, H. N., J. Am. Chem. Soc. 77, 1273 (1955))

Die in der Regel mit Dinitrobenzolsulfonat oder Dinitrofluorbenzol durchgeführte Reaktion führt zur Umsetzung der freien Aminogruppen des Proteins unter Bildung von Dinitrophenylderivaten. Auf diese Weise wird dinitrophenyliertes $PP_5$ erhalten.

f) Reaktion mit gemischten Anhydriden
(Literatur: kabat and Mayer's Experimental Immunochemistry, sec. edition 1961, 813—815)

Dieses Verfahren ist geeignet, um eine Reihe verschiedener Haptene, die zunächst durch Acylierung in ein gemischtes Anhydrid überführt werden müssen, mit Aminogruppen des Proteins zu verknüpfen. Zudem lassen sich durch direkte Umsetzung von Proteinen mit Anhydriden in bekannter Weise Säureamide der allgemeinen Formel R—CO—NH-Protein herstellen, wobei R ein beliebiges Hapten sein kann. Mit Hilfe dieser Reaktion läßt sich beispielsweise die $PP_5$-Hapten-Verbindung mit R=Benzyl als Hapten herstellen.

g) Reaktion mit Carbodiimiden
(Literatur: Makino, T. et. al., Contraception 8 (2), 133 (1973))

Die Carbodiimide der allgemeinen Formel R—N=C=N—R (R=ein beliebiger organischer Rest) sind geeignet, um Carboxyl-Gruppen enthaltende Haptene mit Aminogruppen von Proteinen zu verknüpfen. Auch Hierbei wird als Reaktionsprodukt die Verbindung R—CO—NH-Protein erhalten, wobei R jedes beliebige Hapten, das eine Carboxylgruppen besitzt, sein kann. Mit Hilfe dieser Reaktion wird beispielsweise die Hapten-Verbindung des $PP_5$ mit Cyclohexancarbonsäure als Hapten erhalten.

Das immunologische Verhalten des $PP_5$ läßt sich erfindungsgemäß auch durch Verknüpfung des Glykoproteins mit einem Peptid oder Protein verändern. Die Verknüpfung kann entweder kovalent oder durch Komplexbildung vorgenommen werden. Als Reaktionspartner sind hier Peptide, beispielsweise auch solche mit eigener biologischer Funktion, verwendbar, z.B. Dekapeptide wie der LRF (Luteinisierendes Hormon Relasing Factor). Der LRF ist beispielsweise in der gleichen Weise mit dem $PP_5$ zu verknüpfen wie dies für dessen Verknüpfung mit Albumin von T. Makino u.a. in Contraception 8, 2 (1973), S. 133 ff., beschrieben ist. Die zugrundeliegende Reaktion geht auf die Carbodiimid-Aktivierung der Carboxylgruppen mit Carbodiimid-Verbindungen zurück.

Eine weitere Möglichkeit zur Veränderung des immunologischen Verhaltens des $PP_5$ besteht in der Verknüpfung des Proteins mit einer zweiten Proteinsubstanz in der Weise, wie dies beispielsweise für die Verknüpfung von Enzymen mit Proteinen bekannt ist, wobei das $PP_5$ in der Reaktion einen der beiden Partner ersetzen kann. Für die Verbindung der beiden Proteine miteinander sind eine Reihe von

Verfahren geläufig. Sie verwenden einzelne Gruppen von aktivierenden Substanzen wie beispielsweise Carbodiimide, Cyanurchlorid, p,p-Diflour-m,m-dinitrophenylsulfon oder Glutardialdehyd. Das $PP_5$ kann z.B. mit Hilfe von Glutardialdehyd in einer Reaktion analog der, die von S. Avrameas in Immunochemistry *6*, (1969), 43—52, für die Kopplung von Gammaglobulin mit Peroxydase beschrieben ist, mit einer Reihe von Proteinen durchgeführt werden. Vorzugsweise wird im Sinne der vorliegenden Erfindung das als immunisierendes Agens vorgesehene $PP_5$ mit einer Proteinsubstanz verbunden, die selbst als Impfstoffkomponente wirksam ist, wie Tetanustoxoid. Andere geeignete Träger für das $PP_5$ sind beispielsweise Flagellin, Hämocyanin, Dextran, abgetötete Viren oder Bakterien, deren Toxoide sowie synthetische Polypeptide und Polynucleotide und schließlich biologisch abbaubare Polymere wie Polymilchsäure, Polyglykolsäure oder Kollagen.

Eine bevorzugte Ausführungsform hierfür stellt das Reaktionsprodukt von $PP_5$ mit Tetanustoxoid dar, welche darin kovalent in einem Molverhältnis von etwa 10:1 bis 1:1 z.B. mit Hilfe von Glutardialdehyd verbunden sind.

Es gehören zum Gegenstand der Erfindung Verfahren zur Herstellung von $PP_5$-Derivaten, d.h. pro-teinmodifizierten Verbindungen von $PP_5$, das durch Extraktion von menschlicher Plazenta erhalten worden und zur Proteinmodifizierung einem Verfahren zur Oxydation, Reduktion, Alkylierung oder Acylierung von Proteinen, zur Herstellung einer kovalenten Bindung zwischen Protein und Haptenen oder zwischen Proteinen untereinander unterworfen worden ist.

Die erhaltenen Reaktionsprodukte werden entsprechend bekannten Techniken in die für die parenterale Injektion geeignete Zubereitung überführt und wie bei Impfstoffen üblich ggf. mit stabilisierenden Zusätzen und anorganischen oder organischen Immunadjuvantien versehen, um auf diese Weise als aktive immunisierendes Agens für die Geburtenkontrolle eingesetzt werden zu können.

Als stabilisierende Zusätze kommen Stoffe in Frage, welche die Haltbarkeit der Präparate verbessern, insbesondere niedrig-molekulare Kohlenhydrate oder Eiweißstoffe oder deren Derivate wie abgebautes und wiedervernetztes Kollagen, beispielsweise das unter dem Warenzeichen Haemaccel[R] erhältliche Gelatine-Produkt, ggf. mit weiteren Zusätzen wie Aminosäure-Salzen, z.B. Natriumglutaminat. Die Menge derartiger Zusätze beträgt zweckmäßig 0,5% bis 5%, zuweilen bis zu 10%. Das immunisierende Agents kann danach flüssig oder in trockener, zweckmäßig in gefriergetrockneter Form, zur Verfügung gestellt werden. Letzteres wird vor der Applikation im Wasser oder einem physiologisch verträglichen Medium aufzulösen sein.

Für die wirksame Dosis und deren Verabreichung gelten zwei grundsätzlich verschiedene Über-legungen. Werden direkt mit $PP_5$ reagierende Antikörperpräparationen parenteral verabreicht, genügt häufig eine einmalige Injektion von etwa 50 bis 500 mg Antikörpereiweiß pro kg Körpergewicht. Bei den Präparationen, mit welchen eine Antikörperbildung gegen das $PP_5$ induziert werden soll, verwendet man mehrfach Einzeldosen von etwa 0,1 bis 50 mg. Diese wird zweckmäßigerweise 1 bis 5 Mal in Intervallen von 1—3 Wochen verabreicht.

Die folgende Versuchsbeschreibung zeigt den Erfolg der passiven sowie aktiven Immunisierung von Affen mit $PP_5$-Antikörpern bzw. Derivaten des $PP_5$.

1. Unterbrechung der Schwangerschaft durch passive Immunisierung

A. Gewinnung von Antiseren und Reinigung der Antikörper. 40 Kaninchen werden mit menschlichem $PP_5$ immunisiert

Dazu wird das $PP_5$ oder dessen Derivat in physiologischer Kochsalzlösung gelöst (Konzentration 0,06 mg/3 ml) und unter Zusatz von Aluminiumhydroxid zu einer Suspension verrührt. Die Kaninchen erhalten an 5 aufeinanderfolgenden Tagen je 0,05 mg Protein in 3 ml Suspension pro Tier i.v. injiziert. Anschließend läßt man die Tiere 10 Tage ruhen.

Dann immunisiert man wieder an 5 aufeinanderfolgenden Tagen mit der oben angegebenen gleichen Menge Antigen, läßt die Tiere wieder 10 Tage ruhen und injiziert schließlich nochmals an 5 aufeinderfolgenden Tagen je 0,05 mg des Antigens. Nach einer erneuten Wartezeit von 7 bis 9 Tagen werden die Tiere entblutet. Nach dem Gerinnen des Blutes wird das serum vom Blutkuchen abgeschleudert und gewonnen.

Aus dem Serum der Tiere werden die Antikörper durch Fällung mit $(NH_4)_2SO_4$ (30% w/v) und durch Chromatographie mittels DEAE-Cellulose und 0,03 molarem Phosphatpuffer pH 7,0 isoliert. Die Antikörper wandern dabei ungehindert durch die Säule, die übrigen Serumproteine bleiben an die DEAE-Cellulose gebunden. Das Eluat mit den Antikörpern wird auf 5% Eiweiß ankonzentriert, mit 2,25% Glycin versetzt, sterilfiltriert, in Portionen zu 5 ml abgefüllt und lyophilisiert. Jede Abfüllung enthält 250 mg Eiweiß.

B. Unterbrechung der Schwangerschaft

Trächtige Affen der Gattung Cynomolgus erhielten zwischen dem 21. und 27. Schwangerschaftstag an drei aufeinanderfolgenden Tagen je 250 mg des Antikörper-Präparates aus dem Kaninchen-serum gegen $PP_5$ in 5 ml gelöst intravenös verabreicht. 5 von 7 dieser passiv immunisierten Tiere abortierten zwischem dem 33. und 48. Tage der Schwangerschaft. Ein anderer Affe (Nr. 76) hatte eine Totgeburt am 128. Tag. Nur 1 Affe (Nr. 83) trug seine Schwangerschaft normal aus (vgl. Tabelle I).

Tabelle I
Passive Immunisierung mit $PP_5$

| Cynommolgus Affe | Applikation der $PP_5$-Antikörper Tage der Schwangerschaft | Abort Tag der Schwangerschaft | Geburt |
|---|---|---|---|
| 75 | 21—23 | 33 | nein |
| 76 | 25—27 | 128 | ja |
| 77 | 24—26 | 48 | nein |
| 78 | 24—26 | 38 | nein |
| 81 | 25—27 | 44 | nein |
| 82 | 23—25 | 35 | nein |
| 83 | 24—26 | — | ja |

2. Konzeptionsverhütung durch aktive Immunisierung mit Derivaten des $PP_5$

Geschlechtsreife weibliche Affen (Cynomolgen) wurden über einen Zeitraum von 6 Wochen mit insgesamt 3 mg eines Derivates von $PP_5$ in isotonischer Kochsalzlösung und fein verteiltem Aluminiumhydroxyd als Adjuvans abwechselnd intravenös und subkutan immunisiert. Nach der Immunisierung wurden die Tiere jeweils zum Zeitpunkt der Ovulation für 3 Tage mit einem befruchtungsfähigen männlichen Tier zur Paarung zusammengebracht und dann das Ergebnis der Kopulation registriert. Die gegen $PP_5$ immunisierten weiblichen Affen waren gegenüber nichtbehandelten Kontrolltieren in ihrer Fortpflanzungsfähigkeit stark gehemmt. Das Ergebnis bei den immunisierten Tieren war wie folgt:

Von den 11 Affen, welche mit $PP_5$-Präparationen immunisiert worden waren, wurden nur 2 nach der ersten Kopulation trächtig. Ein weiterer Affe konzipierte erst nach der 2. Kopulation, 3 nach der dritten und einer nach der 4. Kopulation. Von den insgesamt 7 trächtig gewordenen Affen haben bereits 2 wieder abortiert.

Die Kontrollgruppe bestand aus 5 Tieren, die in der gleichen Weise wie die übrigen Tiere gehalten wurden. Sie erhielten jedoch statt der $PP_5$-Präparation lediglich ein isotonische Kochsalzlösung mit fein verteiltem Aluminiumhydroxid. In dieser Gruppe wurden 3 Affen nach der 1. Kopulation trächtig, ein weiteres Tier nach der 2. und das letzte nach der 4. Kopulation. Alle Tiere brachten gesunde Junge zur Welt.

Die nachfolgenden Beispiele dienen zur weiteren Erläuterung der Erfindung.

Beispiel 1:
Modifizierung von $PP_5$ mit diazotierter Sulfanilsäure
a) Herstellung des Diazoniumsalzes

1 g Sulfanilsäure werden bei Zimmertemperatur in 100 ml 0,1N HCl gelöst, dann in Eiswasser gestellt und unter Rühren auf 0°C abgekühlt. Die Sulfanilsäure fällt dabei als Salz aus. In die Suspension läßt man dann 50 ml einer kalten 1%igen Lösung von Natriumnitrit in Wasser langsam (im Verlauf von 1 Stunde) unter Rühren eintropfen; das Ende der Reaktion wird durch die Blaufärbung von Kaliumjodid-Stärkepapier angezeigt.

b) Herstellung der Hapten-Verbindung

Die Konjugation von $PP_5$ mit der diazotierten Sulfanilsäure erfolgt bei schwach alkalischem pH. Man löst dazu 20 mg $PP_5$ in 2 ml eines 0,2 M Natriumphosphatpuffers von pH 8,2 und kühlt die Lösung auf 4°C ab. Dann gibt man 0,11 ml der Diazoniumsalzlösung hinzu und rührt 4 Stunden bei 4°C. Der pH-Wert, der nicht unter 8,0 absinken soll, wird dabei durch Zugabe von 0,2 N NaOH korrigiert. Anschließend läßt man die Lösung 15 Stunden bei 4°C stehen. Dan modifizierte Protein wird dann mehrere Tage gründlich gegen Wasser dialysiert und schließlich lyophilisiert.

Eine Dosis des immunisierenden Agens hat folgende Zusammensetzung: 0,2 mg des Derivates von $PP_5$ gelöst in 3 ml isotonischer Kochsalzlösung enthaltend 0,05% Aerosil, 5% Haemaccel[R] und als Konservierungsmittel 0,15 mg Natriumtimerfonat.

Beispiel 2:
Modifizierung von $PP_5$ durch Kupplung mit Tetanustoxoid

Die kovalente Verknüpfung von $PP_5$ mit Tetanustoxoid wird in wäßriger Lösung bei pH 5,0 unter Verwendung eines wasserlöslichen Carbodiimids durchgeführt:

20 mg $PP_5$ und 60 mg Tetanustoxoid

20 mg $PP_5$ und 60 mg Tetranustoxoid werden in 5,8 ml Wasser gelöst, durch Zugabe von 1/10 n Salzsäure auf pH 5,0 eingestellt und dann unter Rühren mit 6,6 mg N-Äthyl-N'-(3-Dimethylamino-

6

propyl)-Carbodiimid-HCl versetzt. Das Gemisch wird 3 Stunden bei Zimmertemperatur (20°C) und anschließend 20 Stunden bei +4°C gerührt, dann neutralisiert und gegen isotone Kochsalzlösung dialysiert.

Eine Dosis des immunisierenden Agens hat folgende Zusammensetzung:

0,5 mg Protein in 3 ml isotonischer Kochsalzlösung und 1,5 mg Al(OH)$_3$ oder ein anderes immunologisches Adjuvans).

**Patentansprüche**

1. Mittel zur Konzeptionsverhütung und Schwangerschaftsunterbrechung bei Primaten, dadurch gekennzeichnet, daß es eine wirksame Menge einer Substanz enthält, welche bei parenteraler Verabreichung mit dem Protein PP$_5$ immunologisch zu reagieren oder immunologisch reaktive Substanzen, die gegen das PP$_5$ gerichtet sind, zu induzieren vermag.

2. Mittel nach Anspruch 1, dadurch gekennzeichnet, daß es gegen das PP$_5$ gerichtete Antikörper enthält.

3. Mittel nach Anspruch 1, dadurch gekennzeichnet, daß es PP$_5$ oder dessen Derivative enthält.

4. Verfahren zur Herstellung von Derivaten des PP$_5$, dadurch gekennzeichnet, daß das PP$_5$ einem an sich bekannten Verfahren zur Oxydation, Reduktion, Alkylierung oder Acylierung von Proteinen, zur Herstellung einer kovalenten Bindung zwischen Protein und Hapten oder zwischen Proteinen untereinander unterworfen wird.

5. Verfahren zur Herstellung eines Mittels nach Anspruch 2, dadurch gekennzeichnet, daß man Tieren eine wirksame Menge des PP$_5$ parenteral verabreicht, nach Ausbildung von gegen PP$_5$ gerichteten Antikörper Blut von den immunisierten Tieren gewinnt, daraus das Serum und gegebenenfalls die antikörperreiche Fraktion gewinnt und diese in eine für die parenterale Applikation geeignete Zubereitungsform bringt.

6. Verfahren zur Herstellung eines Mittels nach Anspruch 3, dadurch gekennzeichnet, daß das PP$_5$ einem an sich bekannten Verfahren zur Oxydation, Reduktion, Alkylierung oder Acylierung von Proteinen, Zur Herstellung einer kovalenten Bindung zwischen Protein und Hapten oder zwischen Proteinen untereinander unterworfen und danach in eine für die parenterale Applikation geeignete Zubereitung gebracht wird.

**Revendications**

1. Agent pour la contraception et l'interruption de la grossesse chez les primates, caractérisé en ce qu'il contient une quantité efficace d'une substance qui, par administration parentérale, peut réagir immunologiquement avec la protéine PP$_5$ ou induire des substances immunologiquement réactives qui sont opposées à la PP$_5$.

2. Agent selon la revendication 1, caractérisé en ce qu'il contient des anticorps opposés à la PP$_5$.

3. Agent selon la revendication 1, caractérisé en ce qu'il contient de la PP$_5$ ou ses dérivés.

4. Procédé de préparation de dérivés de la PP$_5$, caractérisé en ce que la PP$_5$ est soumise à un procédé connu en soi pour l'oxydation, la réduction, 1-alkylation ou l'acylation des protéines, pour la préparation d'une liaison covalente entre protéine et haptène ou entre protéines.

5. Procédé de préparation d'un agent selon la revendication 2, caractérisé en ce qu'on administre à des animaux par voie parentéale une quantité efficace de la PP$_5$, après formation des anticorps opposés à la PP$_5$ on prélève le sang des animaux immunisés, dont on récupère le sérum et éventuellement la fraction riche en anticorps et on la met sous une forme appropriée pour l'administration parentérale.

6. Procédé de préparation d'un agent selon la revendication 3, caractérisé en ce que la PP$_5$ est soumise à un procédé connu en soi pour l'oxydation, la réduction, l'alkylation ou l'acylation des protéines, pour la préparation d'une liaison covalente entre protéine et haptène ou entre protéines, puis est mise sous une forme appropriée pour l'administration parentérale.

**Claims**

1. Agent for the prevention of conception and interruption of pregnancy in primates, characterized by a content of an effective amount of a substance which, upon parenteral administration, immunologically reacts with the proteins PP$_5$ or induces immunologically reactive substances directed against PP$_5$.

2. An agent as claimed in Claim 1, containing antibodies directed against PP$_5$.

3. An agent as claimed in Claim 1, containing PP$_5$ or its derivatives.

4. A process for the preparation of derivatives of PP$_5$, which comprises subjecting PP$_5$ to a process for the oxidation, reduction, alkylation or acylation of proteins to produce a covalent linkage between protein and haptene or between proteins themselves.

5. A process for the preparation of an agent as claimed in Claim 2, wherein an effective amount of PP$_5$ is administered parenterally to animals, after formation of antibodies directed against PP$_5$, blood is

withdrawn from the immunised animals, the serum or the antibody-rich fraction is isolated therefrom and brought into a form suitable for parenteral administration.

6. A process for the preparation of an agent as claimed in Claim 3, wherein $PP_5$ is subjected to a process for the oxydation, reduction, alkylation or acylation of proteins, to produce a covalent linkage between protein and haptene or between proteins themselves and then brought into a form suitable for parenteral administration.